# EUROPEAN PATENT APPLICATION

(11) **EP 2 389 933 A1**
(43) Date of publication of application: **30.11.2011**
(21) Application number: 11167295.2
(22) Date of filing: 24.05.2011
(51) Int. Cl.: A61K 31/197, A61K 9/20, A61K 9/00

(54) **Controlled-Release Pregabalin Compositions**

(30) Priority: 25.05.2010 TR 201004139
(71) Applicant: Sanovel Ilac Sanayi ve Ticaret A.S., 34398 Istanbul (TR)
(72) Inventor: Cifter, Ümit, 34398, Istanbul (TR); Türkyilmaz, Ali, 34398, Istanbul (TR); Akalin, Nur PEHLIVAN, 34398, Istanbul (TR); Yildirim, Aylin, 34398, Istanbul (TR)
(74) Representative: Sevinç, Erkan

(57) **Abstract**

The present invention provides an orally-administrable controlled-release formulation, comprising pregabalin or a pharmaceutically acceptable salt of pregabalin and polycarbophil, as a controlled-release agent.

## Description

### Field of Invention

The present invention relates to pharmaceutical compositions of pregabalin or a pharmaceutically acceptable salt thereof. The present invention more particularly relates to a stable mucoadhesive controlled-release formulation of pregabalin, with a release profile of desired efficiency.

### Background of Invention

Pregabalin is an analog of gamma-aminobutyric acid (GABA). Its chemical designation is (S)-3-(aminomethyl)-5-methyl hexanoic acid, with the chemical structure illustrated below with Formula 1.

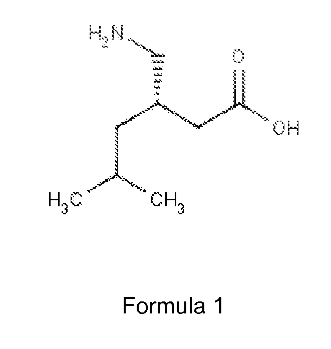

It is known that pregabalin binds to the auxiliary subunit of voltage-sensitive calcium channels in the central nervous system, thereby replacing [3H]-gabapentin. It also reduces the release of many neurotransmitters, including pregabalin glutamate, noradrenaline, and the substance P. It is used for the treatment of epilepsy, simple or complex partial convulsion, either accompanied or not by secondary generalized convulsions, and of neuropathic pain. Various formulations of pregabalin have been developed. It is generally provided in the form of conventional capsule formulations. Posology requires such formulations to be administered twice or thrice daily.

Various applications can be found in relation to pregabalin in the patent literature.

Patent application WO2008008120, for instance, discloses a solid dosage form comprising a compacted fill material containing at least one active agent and at least one of a disintegrating agent and wetting agent.

Patent application WO2007052125 claims a pharmaceutical composition containing pregabalin or a pharmaceutically acceptable complex, salt, solvate, or hydrate thereof, and excipients such as a matrix forming agent and a swelling agent.

Patent application WO2008140459, in turn, discloses a solid dosage form comprising a compacted fill material having a pressure-sensitive multi-particulate and at least one cushioning agent. Said multi-particulate and/or cushioning agent comprises at least one active agent and display(s) a weight loss less than 1% in 30 minutes according to the friability test USP 29 test # 1216. The compacted fill material has a density of at least 0.5 g/ml and a tensile strength of less than 0.9 MPa.

Patent application WO2008140460, on the other hand, claims a solid dosage form comprising a compacted fill material including at least one active agent. The solid dosage form displays a weight loss of less than 1 % in 30 minutes in accordance with the friability test USP 29 test # 1216. Compacted fill material particles contain at least one active agent in the matrix and provide a controlled release of the active agent.

Patent application WO2008128775 claims a solid pharmaceutical composition comprising pregabalin as an active agent, together with one or more excipients. Said composition is free from saccharides and comprises no further amino acids.

Patent application WO2009066325 provides a controlled release formulation comprising pregabalin or a pharmaceutically acceptable salt thereof, a hydrophobic release agent, and an excipient.

Stability-related problems do occur in a plurality of active agents, including pregabalin, under the influence of ambient and physical conditions. As disclosed in patent WO9959573, pregabalin, an amino acid derivative, undergoes cyclization and converts into a lactam form, even if normal storage conditions are provided. This is not desirable for the formulation.

Pregabalin is an active agent with quite good solubility and dissolution rate. This fact leads to fluctuations in the release profile of controlled-release formulations aimed to be developed. Such fluctuations, in turn, brings about imbalances in the blood plasma levels of active agent, and resultantly, undesired effects are produced on the subject.

In result, the aforesaid drawbacks require a novelty in the art of pregabalin formulations with antiepileptic, analgesic, and anxiolytic activities.

### Object and Brief Description of Invention

The present invention relates to an easily-administrable controlled-release mucoadhesive pregabalin formulation, eliminating all aforesaid problems and brining additional advantages to the relevant prior art.

Accordingly, the main object of the present invention is to obtain a stable formulation with antiepileptic, analgesic, and anxiolytic activities.

Another object of the present invention is to provide a pregabalin formulation administered orally once or twice per day.

A further object of the present invention is to ensure an high absorption by retarding the advancement of formulation through the gastrointestinal tract, thanks to the mucoadhesive pregabalin formulation which is retained at the stomach.

Yet a further object of the present invention is to provide a stable formulation by preventing pregabalin of the subject formulation from converting into the lactam form via cyclization.

Still a further object of the present invention is to embody a controlled-release formulation with uniform release profile.

An orally-administered controlled-release formulation has been developed to carry out any objects, referred to above and to emerge from the following detailed description.

In a preferred embodiment according to the present invention, said novelty is carried out with pregabalin or a pharmaceutically acceptable salt thereof, and polycarbophil, a controlled-release agent.

In a preferred embodiment according to the present invention, said controlled-release agent used may further comprise at least one or a mixture of carrageen, carbopol, sodium alginate, polyethylene glycol, glyceryl monostearate, guar gum, pectin, resin and glyceryl behenate, with carrageen and/or carbopol being preferred.

In a preferred embodiment according to the present invention, the ratio of pregabalin to the total weight of polycarbophil, carrageen, and carbopol is between 0.01 to 10, preferably 0.1 to 10, and more preferably 0.1 to 6.

In a preferred embodiment according to the present invention, excipients can be used, comprising at least one or a mixture of fillers, glidants, lubricants.

In a preferred embodiment according to the present invention, said fillers comprise at least one or a mixture of lactose, starch, pregelatinized starch, microcrystalline cellulose, mannitol, dicalcium hydrogen phosphate dihydrate, calcium hydrogen phosphate anhydrate, calcium phosphate trihydrate, potassium chloride, silicium dioxide and glucose, with dicalcium hydrogen phosphate dihydrate and/or calcium hydrogen phosphate anhydrate being preferred.

In a preferred embodiment according to the present invention, said glidants comprise at least one or a mixture of colloidal silicone dioxide, talc, aluminum silicate, magnesium silicate, with talc being preferred.

In a preferred embodiment according to the present invention, said lubricant comprises magnesium stearate.

In a preferred embodiment of the present invention, said formulation is mucoadhesive.

Another embodiment according to the present invention provides a method for preparing said pharmaceutical formulation, this method comprising the steps of
a. sieving and mixing together pregabalin, polycarbophil, carrageen, carbopol, and dicalcium hydrogen phosphate dihydrate,
b. performing wet granulation process thereon,
c. drying and then sieving wet granules obtained,
d. admixing talc into granules obtained,
e. admixing finally magnesium stearate into the mixture,
f. compressing the resulting mixture into tablets or filling it into capsules.

A further embodiment according to the present invention provides a method for preparing said pharmaceutical formulation, this method comprising the steps of
a. sieving and mixing together pregabalin, polycarbophil, carrageen, carbopol, and dicalcium hydrogen phosphate dihydrate,
b. carrying out dry granulation process thereon and sieving the granules obtained,
c. admixing talc into granules obtained,
d. admixing finally magnesium stearate into the mixture,
e. compressing the resulting mixture into tablets or filling it into capsules.

Another embodiment according to the present invention provides a method for preparing said pharmaceutical formulation, this method comprising the steps of
a. sieving and mixing together pregabalin, polycarbophil, carrageen, carbopol, and calcium hydrogen phosphate anhydrate and talc,
b. admixing magnesium stearate into the powder mixture obtained, and continuing to mix it,
c. compressing the resulting mixture into tablets or filling it into capsules.

In a preferred embodiment according to the present invention, said pharmaceutical formulation is consist of
a. pregabalin or a pharmaceutically acceptable salt thereof at 5-85% by weight
b. polycarbophil at 2-30% by weight
c. carrageen at 5-80% by weight
d. carbopol at 2-50% by weight
e. dicalcium hydrogen phosphate dihydrate and calcium hydrogen phosphate anhydrate at 2-85% by weight,
f. talc at 0.1-5% by weight
g. magnesium stearate at 0.1-5% by weight.

### Detailed Description of Invention

### Example 1

| **Ingredient** | **amount %** |
|---|---|
| pregabalin | 50-75 |
| polycarbophil | 5-20 |
| filler | 5-40 |
| glidant | 0.75-2 |
| lubricant | 0.75-2 |

Various production methods can be applied for the mucoadhesive controlled-release formula of pregabalin with the formulation indicated above.

In the wet granulation production, pregabalin, polycarbophil, and the filler dicalcium hydrogen phosphate dihydrate are sieved and mixed together. Then wet granulation is performed and wet granules obtained are dried and sieved. Into the granules obtained are first added talc, and finally magnesium stearat and mixing is continued. Finally, the mixture is compressed into tablets, or filled into capsules.

Other excipients can be used except those indicated in the example, whereas other production methods are applicable as well. Particularly at least one, a part, or proper mixtures of polycarbophil, carrageen, carbopol can be used as excipients, as shown below.

In the wet granulation production, pregabalin, polycarbophil, carrageen, carbopol and dicalcium hydrogen phosphate dihydrate are sieved and mixed together. Then wet granulation is performed and wet granules obtained are dried and sieved. Into the granules obtained are first added talc, and finally magnesium stearat and mixing is continued. Finally, the mixture is compressed into tablets, or filled into capsules.

In the dry granulation production pregabalin, polycarbophil, carrageen, carbopol, and dicalcium hydrogen phosphate dihydrate are sieved and mixed together. Dry granulation is performed and the granules obtained are sieved. Into the granules obtained are first added talc, and finally magnesium stearat and mixing is continued. Finally, the mixture is compressed into tablets, or filled into capsules.

According to another method, pregabalin, polycarbophil, carrageen, carbopol, and calcium hydrogen phosphate anhydrate and talc are sieved and mixed together. Dry granulation is performed and the granules obtained are sieved. Into the granules obtained is added magnesium stearat and mixing is continued. Finally, the mixture is compressed into tablets, or filled into capsules.

### Example 2

| **Ingredient** | **amount %** |
|---|---|
| pregabalin | 41,25 |
| polycarbophil | 5 |
| carbomer | 5 |
| hydroxypropyl methyl cellulose | 5 |
| dibasic calcium phosphate dihydrate | 42,05 |
| Colloidal silicone dioxide | 0,2 |
| Magnesium stearate | 1,5 |
| **Film coating** | |
| Opadry | 3-5 |

In the wet granulation production, pregabalin, polycarbophil, hydroxypropyl methyl cellulose, carbomer (%4) and dibasic calcium phosphate dihydrate are sieved and mixed together. Then wet granulation is performed with water and carbomer (%1) .Wet granules are dried. Then granules are added colloidal silicone dioxide and mixed and sieved. Into the final mixture is added magnesium stearate and then are mixed. Finally, the mixture is compressed into tablets and coated.

### Example 3

| **Ingredient** | **amount %** |
|---|---|
| pregabalin | 41,25 |
| polycarbophil | 30 |
| Microcrystalline cellulose | 5 |
| dibasic calcium phosphate dihydrate | 22,05 |
| Colloidal silicone dioxide | 0,2 |
| Magnesium stearate | 1,5 |

| **Film coating** | |
|---|---|
| Opadry | 3-5 |

In the direct compression production, pregabalin, polycarbophil, microcrystalline cellulose and dicalcium hydrogen phosphate dihydrate are sieved and mixed together. Then mixture is compacted. Then granules are added colloidal silicone dioxide and mixed and sieved. Into the result mixture is added magnesium stearate and then are mixed. Finally, the mixture is compressed into tablets and coated.

### Example 4

| **Ingredient** | **amount %** |
|---|---|
| pregabalin | 41,25 |
| polycarbophil | 10 |
| Lactose monohydrate | 23.525 |
| dibasic calcium phosphate dihydrate | 23,525 |
| Colloidal silicone dioxide | 0,2 |
| Magnesium stearate | 1,5 |

| **Film coating** | |
|---|---|
| Opadry | 3-5 |

In the wet granulation production, pregabalin, polycarbophil, lactose monohydrate and dibasic calcium phosphate dihydrate are sieved and mixed together. Then wet granulation is performed with water. Wet granules are dried. Then granules are added colloidal silicone dioxide and mixed and sieved. Into the final mixture is added magnesium stearate and then are mixed. Finally, the mixture is compressed into tablets and coated.

This invention has surprisingly provided a controlled-release mucoadhesive pregabalin tablet formulation, which is stable and has a desired release profile. The system according to the present invention adheres to gastric mucosa, so that the passage of tablet through the gastrointestinal tract is retarded. Thus, the advancement of the pregabalin-containing formulation through the gastrointestinal tract is delayed and its absorption is made to occur at a site in which adsorption is more efficient. Absorption of pregabalin can only occur at a certain site of small intestine. Retaining the drug upstream of the efficient absorption site enhances the bioavailability of drug. Polycarbophil provided excellent bioadhesive properties and controlled release properties. Polycarbophil has bioadhesive properties and good binding characteristics . Polycarbophil are efficient matrix forming excipients. These polymers are not soluble, but only swellable in water. Unlike linear polymers, polycarbophil do not dissolve during the release process.

It is possible to make various controlled-release formulations containing pregabalin. It is possible, for instance, to develop extracorporeally-solid tablets, providing swelling gels with increasing volume in stomach, or extracorporeally-liquid formulation, providing the same. These systems, for instance, can float within stomach due to their low densities. Various excipients can be used for this purpose, such as alginates (salts thereof), gums, oils, gelling agents.

The present invention is used for treating epilepsy, pain, anxiety, diabetic neuropathy, neuropathic pain, partial seizure, social phobia, postherpetic neuralgia.

It is further possible to use the following additional excipients in the formulation.

The pharmaceutical compositions according to the present invention may also comprise one or more pharmaceutically acceptable excipients. Such pharmaceutically acceptable excipients include, but are not restricted to fillers, binders, glidants, lubricants, disintegrants, surface active agents, preserving agents, coating agents etc., and the mixtures thereof.

Suitable binders include, but are not restricted to, at least one or a mixture of polyvinylprolidone, gelatin, sugars, glucose, natural glue, gums, synthetic celluloses, polymethacrylate, hydroxypropyl methyl cellulose, hydroxypropyl cellulose, carboxymethyl cellulose, methyl cellulose, and other cellulose derivatives.

Suitable lubricants include, but are not restricted to, at least one or a mixture of sodium stearil fumarat, polyethylene glycol, stearic acid, metal stearates, boric acid, sodium chloride benzoate and acetate, sodium or magnesium lauryl sulfate.

Suitable preservatives include, but are not restricted to, at least one or a mixture of methyl paraben and propyl paraben and salts thereof (e.g. sodium or potassium salts), sodium benzoate, citric acid, benzoik acid, butylated hydroxytoluene and butylated hydroxyanisole.

Suitable surface active agents include, but are not restricted to, at least one or a mixture of sodium lauryl sulfate, dioctyl sulfosuccinate, polysorbates and polyoxyethylene alkyl esters and ethers thereof, glyceryl monolaurate saponins, sorbitan laurate, sodium lauryl sulfate, magnesium lauryl sulfate.

Suitable coating agents include, but are not restricted to hydroxypropyl methyl cellulose, polyethylene glycol, polyvinylprolidone, polyvinylprolidone-vinyl acetate copolymer (PVP-VA), polyvinyl alcohol like polymers, and all sorts of Opadry™, as well as pigments, dyes, titanium dioxide and iron oxide, talc.

The present invention is hereby disclosed by referring to an exemplary embodiment hereinabove. Whilst this exemplary embodiment does not restrict the object of the present invention, the latter must be assessed under the light of the foregoing detailed description.

## Claims

1. An orally-administrable controlled-release formulation, comprising pregabalin or a pharmaceutically acceptable salt of pregabalin and polycarbophil, as a controlled-release agent.

2. The pharmaceutical formulation according to Claim 1, **characterized by** comprising also at least one or a mixture of carrageen, carbopol, sodium alginate, polyethylene glycol, glyceryl monostearate, guar gum, pectin, resins and glyceryl behenate, with carrageen and/or carbopol being preferred.

3. The pharmaceutical formulation according to any of the preceding claims, wherein the ratio of pregabalin to the total weight of polycarbophil, carrageen, and carbopol is between 0.01 to 10, preferably 0.1 to 10, and more preferably 0.1 to 6.

4. The pharmaceutical formulation according to any of the preceding claims, further containing excipients, comprising at least one or a mixture of fillers, glidants, lubricants.

5. The pharmaceutical formulation according to any of the preceding claims, wherein said fillers comprise at least one or a mixture of lactose, starch, pregelatinized starch, microcrystalline cellulose, mannitol, dicalcium hydrogen phosphate dihydrate, calcium hydrogen phosphate anhydrate, calcium phosphate trihydrate, potassium chloride, silicium dioxide and glucose, with dicalcium hydrogen phosphate dihydrate and/or calcium hydrogen phosphate anhydrate being preferred.

6. The pharmaceutical formulation according to any of the preceding claims, **characterized in that** said glidants comprise at least one or a mixture of colloidal silicone dioxide, talc, aluminum silicate, and magnesium silicate, with talc being preferred.

7. The pharmaceutical formulation according to any of the preceding claims, wherein said lubricant is magnesium stearate.

8. The pharmaceutical formulation according to any of the preceding claims, wherein this formulation is mucoadhesive.

9. A method for preparing a pharmaceutical formulation according to any of the preceding claims, comprising the steps of
a. sieving and mixing together pregabalin, polycarbophil, and dicalcium hydrogen phosphate dihydrate,
b. performing wet granulation process thereon,
c. drying and then sieving wet granules obtained,
d. admixing talc into granules obtained,
e. admixing finally magnesium stearate into the mixture,
f. compressing the resulting mixture into tablets or filling it into capsules.

10. A method for preparing a pharmaceutical formulation according to any of the preceding claims, comprising the steps of
a. sieving and mixing together pregabalin, polycarbophil, and dicalcium hydrogen phosphate dihydrate,
b. carrying out dry granulation process thereon and sieving the granules obtained,
c. admixing talc into granules obtained,
d. admixing finally magnesium stearate into the mixture,
e. compressing the resulting mixture into tablets or filling it into capsules.

11. A method for preparing a pharmaceutical formulation according to any of the preceding claims, comprising the steps of
a. sieving and mixing together pregabalin, polycarbophil, calcium hydrogen phosphate anhydrate and talc,
b. admixing sieved magnesium stearate into the powder mixture obtained, and continuing to mix it,
c. compressing the resulting mixture into tablets or filling it into capsules.

12. The pharmaceutical formulation according to any of the preceding claims , consisting of
a. pregabalin or a pharmaceutically acceptable salt thereof at 5-85% by weight
b. polycarbophil at 2-30% by weight
c. carrageen at 5-80% by weight
d. carbopol at 2-50% by weight
e. dicalcium hydrogen phosphate dihydrate and calcium hydrogen phosphate anhydrate at 2-85% by weight,
f. talc at 0.1-5% by weight
g. magnesium stearate at 0.1-5% by weight.

13. Use of a pharmaceutical formulation according to any of the preceding claims for preventing or treating epilepsy, pain, anxiety, diabetic neuropathy, neuropathic pain, postherpetic neuralgia in mammalians and particularly in humans.
